# EUROPÄISCHE PATENTSCHRIFT

(11) **EP 0 679 438 B1**
(45) Veröffentlichungstag und Bekanntmachung des Hinweises auf die Patenterteilung: **07.04.1999**
(21) Anmeldenummer: 95106338.7
(22) Anmeldetag: 27.04.1995
(51) Int. Cl.: B01J 31/18, C07F 9/142, C07F 9/145, C07C 17/08, C07C 21/04

(54) **Verfahren zur Herstellung von 1-Chlor-3-methyl-but-2-en**
Process for the preparation of 1-chloro-3-methyl-2-butene
Procédé pour la préparation de 1-chloro-3-méthyl-2-butène

(30) Priorität: 28.04.1994 DE 4414964
(43) Veröffentlichungstag der Anmeldung: 02.11.1995
(73) Patentinhaber: Wacker-Chemie GmbH, 81737 München (DE)
(72) Erfinder: Petersen, Hermann, Dr., D-84489 Burghausen (DE); Huebner, Dieter, Dr., D-84489 Burghausen (DE)
(74) Vertreter: Schuderer, Michael, Dr.

(56) Entgegenhaltungen:
- DE-A- 2 538 636
- DE-A- 3 804 265
- US-A- 4 036 899
- J. ORG. CHEM., Bd. 44, Nr. 19, 1979 Seiten 3428-3430, ZIEGLER AND CHAN TAM 'Ketene Dioachetals. The control of the allylation using cuprous salts'
- J. ORG. CHEM, Bd. 46, 1981 Seiten 5200-5204, AXELRAD AND AL. 'Reactions of Copper halide complexes of trivalent Phosphorus with vinylic halides'

## Beschreibung

Die Erfindung betrifft ein Verfahren zur Herstellung von 1-Chlor-3-methyl-but-2-en unter Verwendung von Stoffen, die als Reaktionskatalysatoren, Isomerisierungskatalysatoren und Stabilisatoren bei der Herstellung von 1-Chlor-3-methyl-but-2-en (Prenylchlorid) wirken.

1-Chlor-3-methyl-but-2-en (Prenylchlorid) wird durch Anlagerung von HCl an Isopren und anschließende Isomerisierung des hauptsächlich gebildeten 3-Chlor-3-methyl-but-1-en zu Prenylchlorid dargestellt.

In der DE-A 2538636 (CA 86: 189176) wird die Umsetzung von Isopren mit gasförmigem HCl bei erhöhtem Druck und Temperaturen > 10°C und unter Verwendung von CuCl-Pulver als Katalysator beschrieben. Nachteilig ist, daß aufgrund des festen Katalysators nur eine diskontinuierliche Fahrweise möglich ist, die Durchführung bei dem beschriebenen Druckniveau die Verwendung von aufwendigen Autoklaveneinrichtungen notwendig macht und sehr lange Reaktionszeiten von 6 bis 8 Stunden für die Umsetzung benötigt werden.

Die DE-A 2143095 (CA 77: 4859) betrifft ein Verfahren bei dem die Umsetzung von Isopren mit gasförmigem HCl in Gegenwart von pulverförmigem Kupfer oder CuCl bei sehr niederem Temperaturniveau von unter 0°C durchgeführt wird. Nachteilig ist hier die unwirtschaftlich niedere Reaktionstemperatur sowie die Notwendigkeit, den Katalysator vor der Gewinnung des erwünschten Endprodukts vollständig zu entfernen, da andernfalls Prenylchlorid nur in einem sehr ungünstigen Gleichgewichtsverhältnis von 12 : 88 zu Isoprenylchlorid vorliegt. Eine kontinuierliche Fahrweise verbietet sich damit von selbst.

Katalysatoren für die Isomerisierung von 3-Chlor-3-methyl-but-1-en (Isoprenylchlorid) zu Prenylchlorid werden in der EP-A 132543 und der EP-A 132545 beschrieben. Mit dem Verfahren gemäß der EP-A 132543 erfolgt die Isomerisierung in Gegenwart von gasförmigem Chlorwasserstoff, elementarem Kupfer oder einem Cu(I)- bzw. Cu(II)-Chlorid und einem quartären Ammoniumsalz. Die EP-A 132545 beschreibt die Isomerisierung in Gegenwart von gasförmigem Chlorwasserstoff, elementarem Kupfer oder einem Cu(I)- bzw. Cu(II)-Chlorid und einem organischen Amin.

Ein Nachteil der Isomerisierung mit elementarem Kupfer oder Cu(I)- bzw. Cu(II)-Chlorid ist, daß diese Stoffe im Ausgangs- und Endprodukt praktisch unlöslich sind. Deshalb müssen für eine ausreichende Wirksamkeit große Mengen eingesetzt werden und der Katalysator nach der Umsetzung zusätzlich noch abfiltriert werden. Bei kontinuierlicher Fahrweise ist die Feststoffdosierung technisch problematisch.

Aus einer Reihe von Publikationen sind auch kontinuierliche Verfahren zur Herstellung von Prenylchlorid bekannt. Die CS-A 124000 (CA 69:43403) beschreibt die kontinuierliche Herstellung von Prenylchlorid durch Umsetzung von Isopren mit Chlorwasserstoff bei tiefen Temperaturen in einer Absorptionskolonne und anschließender Trennung des Isomerengemisches in zwei Destillationskolonnen. Dazu wird am Kopf der Kolonne frisches und rückgewonnenes Isopren, rückgewonnenes Isomer sowie Sumpfprodukt aus der ersten Destillation kontinuierlich dosiert und am Boden gleichzeitig Chlorwasserstoff eingeleitet. Wegen der großen Mengen zu recycelnder Isomere bzw. Edukte ist dieses Verfahren wirtschaftlich sehr ungünstig.

In der FR-A 1548516 (CA 71: 112390) wird in Beispiel 2 eine kontinuierliche Fahrweise beschrieben, bei der Isopren mit einem Gemisch aus Salzsäure und Schwefelsäure umgesetzt wird und das so erhaltene Prenylchlorid kontinuierlich abdestilliert wird. Nachteilig ist hierbei der zusätzliche Aufwand durch Mischen von konz. Salzsäure und konz. Schwefelsäure, die Phasentrennung und das Entsorgen der wäßrigen Phase (verdünnte Schwefelsäure).

Gegenstand der US-A 4036899 ist ein Prozeß, bei dem Isopren mit wäßriger HCl in Gegenwart von Alkali- bzw. Erdalkalimetallchloriden umgesetzt wird und das Endprodukt aus der organischen Phase durch Destillation entfernt wird. Nachteilig ist hierbei, daß keine echte kontinuierliche Fahrweise resultiert, da die wäßrige Phase jeweils nach deren Abtrennung verworfen wird.

In der JP-A 50-160206 (CA 84: 135093) wird in eine Mischung aus Isopren und Isoprenylchlorid Chlorwasserstoff eingeleitet und Isopren zu Isoprenylchlorid umgesetzt. Dieses wird mit Chlorwasserstoff oder Salzsäure zu Prenylchlorid isomerisiert. Die isomerisierte Mischung wird schichtengetrennt und die Salzsäurelösung wieder in der Isomerisierungsstufe eingesetzt. Aus der organischen Phase wird nichtumgesetztes Isopren und nichtisomerisiertes Isoprenylchlorid abgetrennt und wieder in die erste Stufe zurückgeführt. Mit dieser Verfahrensweise ist zwar eine kontinuierliche Fahrweise möglich, jedoch ist aufgrund der Verwendung von wäßriger Salzsäure ein aufwendiger Verfahrensschritt zur Schichtentrennung unumgänglich. Nachteilig ist auf jeden Fall die große Gefahr, daß durch Bildung von Lewissäure sehr heftige Polymerisationsreaktionen ausgelöst werden können.

Als Stabilisatoren für Prenylchlorid sind im Stand der Technik N,N-Diarylamine (CA 95: 203290) und t-Alkylamine, Amide und Thioharnstoffe (CA 116: 6147) beschrieben. Kupferhalogenid-Trialkylphosphite sind in Ziegler F. E., J. Org. Chem. 44, 1979, 3428-3430 als Katalysatoren für die Allylierung von Ketendithioacetalen und in Axelrad G., J. Org. Chem. 46, 1981, 5200-5204 als Agentien für die Herstellung von Vinylphosphonaten beschrieben.

Aufgabe der Erfindung war es, Katalysatoren für die Herstellung von Prenylchlorid zur Verfügung zu stellen, mit denen auch die technisch bevorzugte kontinuierliche Darstellung von Prenylchlorid aus Isopren möglich ist. Mit den aus dem Stand der Technik bekannten festen CuCl-Katalysatoren ist dies nicht möglich, da eine laufende Dosierung und Abtrennung des festen CuCl nur unter erheblichem Aufwand zu realisieren ist. Weiterhin sollte die Verfahrenssicherheit, welche in den Verfahren des Stands der Technik nicht berücksichtigt wurde, sichergestellt werden. Bei der Umsetzung von Isopren mit Chlorwasserstoff zu Prenylchlorid reichen nämlich schon sehr geringe Spuren einer Lewissäure wie Eisen(III)chlorid aus, um die spontane Zersetzung von Prenylchlorid zu bewirken und äußerst heftige Polymerisationsreaktionen zu initiieren. Mit den bisherigen Verfahren war selbst in Emailanlagen ein hohes Sicherheitsrisiko gegeben, da durch Beschädigung durchaus Eisen(III)chlorid frei werden kann.

Gegenstand der Erfindung ist ein Verfahren zur Herstellung von 1-Chlor-3-methyl-but-2-en aus Isopren und Chlorwasserstoff in Gegenwart eines Katalysators, dadurch gekennzeichnet, daß als Katalysator ein oder mehrere Komplexe von Kupfer(I)halogenid und Trialkyl- oder Triarylphosphiten der allgemeinen Formel CuX^{·}P(OR)₃ (I), wobei X die Bedeutung Cl, Br oder I hat und R ein geradkettiger oder verzweigter Alkylrest mit 1 bis 18 C-Atomen oder ein Arylrest ist und wobei der Alkyl-und Arylrest gegebenenfalls substituiert sein können, verwendet werden.

Bevorzugte Komplexe der allgemeinen Formel (I) sind solche, in denen X = Cl, Br oder I, und R = Methyl, Ethyl, n-Propyl, iso-Propyl, n-Butyl, iso-Butyl, iso-Amyl, Benzyl oder Phenyl.

Die Herstellung der Kupferhalogenid- Trialkyl(aryl)phosphit-Komplexe ist beschrieben in "Gmelin Cu [B] Lieferung 1, Seite 251, 365, 414. Man gibt beispielsweise CuCl-Pulver in ein Trialkyl(aryl)phosphit und erwärmt, wobei sich das Pulver unter Entfärbung löst. Falls ein Überschuß an Trialkyl(aryl)phosphit verwendet wird, wird eine Lösung des Cu-Komplexes im entsprechenden Trialkyl(aryl)phosphit erhalten.

In einer bevorzugten Ausführungsform bestehen die Katalysatoren aus Lösungen der Komplexe der allgemeinen Formel (I). Geeignete Lösungsmittel sind inerte organische Lösungsmittel wie Ether, beispielsweise Diethylether oder Kohlenwasserstoffe wie Petrolether oder Hexan, Halogenkohlenwasserstoffe, beispielsweise Chloroform oder Ethylbromid, oder aromatische Lösungsmittel wie Toluol. Vorzugsweise werden Lösungen der Kupferhalogenid-Trialkylphosphit- oder -Triarylphosphit-Komplexe im entsprechenden Trialkylphosphit bzw. im entsprechenden Triarylphosphit eingesetzt. Überraschenderweise wurde nämlich festgestellt, daß die als Lösungsmittel eingesetzten Trialkyl- bzw. Triarylphosphite Prenylchlorid bei der Herstellung und der Lagerung stabilisieren. Aus ökonomischen Gründen ist als Katalysator die Lösung von CuCl·P(OEt)₃ in Triethylphosphit besonders bevorzugt.

Die Konzentration der Lösung ist kein limitierender Faktor. Üblicherweise wird die Konzentration so eingestellt, daß die für die Katalyse erforderliche Komplexmenge vorliegt. Bei der Verwendung von Trialkyl- bzw. Triarylphosphit als Lösungsmittel wird die Konzentration so eingestellt, daß sowohl der Katalysator als auch das stabilisierend wirkende Lösungsmittel in den gewünschten Mengen vorliegen.

Der Katalysator CuX^{·}P(OR)₃ wird vorzugsweise in einem Molverhältnis von 0.02 bis 2.0 mMol pro Mol Isopren, besonders bevorzugt in einem Molverhältnis von 0.1 bis 1.5 mMol pro Mol Isopren, eingesetzt. Das angegebene Molverhältnis von 0.02 mMol Katalysator pro Mol Isopren ist die Mindestmenge, mit der noch eine vernünftige Iomerisierungsgeschwindigkeit resultiert. Prinzipiell gibt es nach oben hin keine Grenze, wobei aus wirtschaftlichen Gründen eine möglichst geringe Menge, wie im Rahmen der obengenannten Bereiche, angestrebt wird. Falls eine sehr hohe Isomerisierungsgeschwindigkeit angestrebt wird, sind die obengenannten Maximalwerte von 1.5 mMol bzw. 2.0 mMol Katalysator pro Mol Isopren empfehlenswert.

Werden als Katalysator Lösungen von CuX^{·}P(OR)₃ in P(OR)₃ eingesetzt, wird die Menge von Trialkyl(aryl)phosphit P(OR)₃ in Abhängigkeit von der gewünschten stabilisierenden Wirkung gewählt werden. Hervorragend wirksam sind schon Mengen von 0.03 mMol P(OR)₃ (=0.003 Mol%) pro Mol Isopren. Größere Mengen sind problemlos möglich. Üblicherweise werden 0.002 bis 0.1 Mol%, bezogen auf Isopren, eingesetzt. Bei der Anwesenheit von relativ großen Eisenmengen kann gegebenenfalls mit noch höheren Konzentrationen gearbeitet werden.

Die Herstellung kann sowohl in diskontinuierlicher als auch kontinuierlicher Verfahrensweise durch Umsetzung von Isopren mit gasförmigem Chlorwasserstoff erfolgen. Dazu wird Isopren in leichtem Überschuß oder in äquimolarem Verhältnis zu Chlorwasserstoff eingesetzt. Ein HCl-Überschuß ist zu vermeiden, da sonst der Anteil an unerwünschtem Dichlormethylbutan ansteigt. Bei zu geringen Mengen Chlorwasserstoff sinkt der Isoprenumsatz. Vorzugsweise beträgt das Molverhältnis von HCl zu Isopren von 0.95 : 1 bis 1 : 1.

Die Additionsreaktion von Chlorwasserstoff an Isopren kann bei Temperaturen von -30°C bis +30°C durchgeführt werden. Als geeignetes Temperaturniveau, bei dem die Isoprenverluste niedrig, die Reaktionsgeschwindigkeit aber ausreichend hoch ist, haben sich Temperaturen um 0°C, vorzugsweise von -5°C bis +5°C bewährt. Die Reaktion kann bei Normaldruck oder Überdruck bis zu 5 bar durchgeführt werden.

Nach der Umsetzung wird der Ansatz erwärmt, zweckmäßigerweise auf Raumtemperatur, vorzugsweise auf ein Temperaturniveau von 10°C bis 30°C. Das gebildete Produktgemisch Isoprenylchlorid/Prenylchlorid kann zur Isomerisierung von Isoprenylchlorid zu Prenylchlorid bei dieser Temperatur, bis zur Einstellung des Gleichgewichts, vorzugsweise 0.5 bis 10 Tage, gelagert werden, wobei anschließend der Prenylchlorid-Anteil durch Destillation isoliert wird. Alternativ dazu kann der Prenylchlorid-Anteil unmittelbar anschließend an die Umsetzung destillativ entfernt werden.

Bei der diskontinuierlichen Verfahrensweise wird in einer bevorzugten Ausführungsform der Katalysator zusammen mit dem Isopren vorgelegt, der Ansatz gegebenenfalls abgekühlt und anschließend unter Rühren, gegebenenfalls Kühlen, Chlorwasserstoffgas in den Ansatz eingeleitet. Prinzipiell ist auch eine Verfahrensvariante möglich, bei der nur der Katalysator vorgelegt wird und Isopren sowie Chlorwasserstoff dosiert werden. Bei dieser Verfahrensweise ist allerdings mit stärkerer Nebenproduktbildung zu rechnen. Nach Abschluß der HCl-Einleitung wird der Ansatz erwärmt und das Produkt zur Isomerisierung von Isoprenylchlorid zu Prenylchlorid gelagert. Das Produktgemisch kann gegebenenfalls ohne vorhergehende Lagerung oder nach Lagerung destillativ gereinigt werden. Im Regelfall kann das Produkt nach Lagerung ohne weitere Destillation für die meisten Umsetzungen weiterverwendet werden.

Die bevorzugte Verfahrensweise zur Darstellung von Prenylchlorid aus Isopren und Chlorwasserstoff, unter Verwendung des erfindungsgemäßen Katalysators, ist die kontinuierliche Fahrweise. Dazu kann in einen mit Kreislaufpumpe betriebenen und gekühlten Reaktionskreislauf kontinuierlich eine Isopren/Katalysatormischung oder Isopren und Katalysator getrennt sowie gasförmiger Chlorwasserstoff dosiert werden und gleichzeitig Produktgemisch abgenommen werden. Beim Start der kontinuierlichen Fahrweise ist es besonders günstig, die Anlage mit Reaktionsgemisch als Vorlage zu befüllen. Es kann dabei Isopren oder Katalysator(lösung) getrennt in die Apparatur gegeben werden, vorzugsweise wird der Katalysator oder die Katalysatorlösung zusammen mit Isopren in die Apparatur gegeben und dann lediglich HCl dosiert, bis der gewünschte Umsatz erreicht wird. Ab diesem Zeitpunkt wird dann kontinuierlich Isopren mit Katalysator und HCl dosiert und kontinuierlich Produkt entnommen.

Die kontinuierlich abfließende Produktmischung kann dann ebenfalls, zur Verschiebung oder bis zur Vollendung der Gleichgewichtseinstellung (Prenylchlorid/Isoprenylchlorid = 85/15), gelagert werden und dann destilliert werden. Es ist aber auch möglich, ohne Lagerung, gleich anschließend zur Umsetzung, kontinuierlich zu destillieren und das über den Kopf der Destillationskolonne abdestillierte Isopren/Isoprenylchlorid-Gemisch wieder der Reaktion zuzuführen, wobei das Isopren mit HCl reagieren und das Isoprenylchlorid zu Prenylchlorid isomerisieren kann. Das Sumpfprodukt der Destillationskolonne kann dann gegebenenfalls einer weiteren kontinuierlichen Destillation zugeführt werden, wobei die Abtrennung von Hochsiedern erfolgt und ein Prenylchlorid von hoher Reinheit erhalten wird (Destillation bei 50°C, 120 mbar: 94.1 % Prenylchlorid, 1.8 % Isoprenylchlorid, 0.2 % Isopren).

Das so zugängliche 1-Chlor-3-methyl-but-2-en ist ein wertvolles Zwischenprodukt zur Herstellung von Vitaminen und Duftstoffen. Damit zugängliche Duftstoffe sind beispielsweise Prenylacetat, Prenylbenzoat, Prenylsalicylat, Nerolialdehyd (2,5-Dimethyl-2-vinyl-4-hexanal), Citrowanil (2,5-Dimethyl-2-vinyl-4-hexennitril), Silwanol (2-Ethyl-2-prenyl-3-hexenol) oder Amarocit (1,1-Dimethoxy-2,2-dimethyl-3-phenylpropan).

Mit dem Katalysatorsystem der allgemeinen Formel CuX^{·}P(OR)₃ stehen erstmals Substanzen zur Verfügung, welche sowohl die Anlagerung von Chlorwasserstoff an Isopren, als auch die Umlagerung des zunächst entstehenden 3-Chlor-3-methyl-but-1-en (Isoprenylchlorid) zu 1-Chlor-3-methyl-but-2-en (Prenylchlorid) katalysieren und außerdem in idealer Weise für eine kontinuierliche Produktion geeignet sind, da sie sowohl im Edukt als auch im Produkt löslich sind und schon in geringsten Mengen wirken. Mit den Lösungen von CuX^{·}P(OR)₃ in den entsprechenden Phosphiten P(OR)₃ stehen darüberhinaus Katalysatoren zur Verfügung, welche die Zersetzung und spontane Polymerisation von Prenylchlorid während der Herstellung verhindern und obendrein noch stabilisierend bei der Lagerung von Prenylchlorid wirken. Ein weiterer Vorteil der obengenannten Lösungen besteht darin, daß je nach Konzentration der Lösung, die benötigte Stabilisatorstärke fein eingestellt werden kann.

Die nachfolgenden Beispiele dienen zur weiteren Erläuterung der Erfindung.

### Beispiel 1 bis 6: Herstellung der Katalysatoren

### Beispiel 1:

### CuCl-P(OEt)₃-Komplex in P(OEt)₃ gelöst

19.3 g (0.116 Mol) Triethylphosphit und 0.6 g (0.006 Mol) CuCl wurden in einem Kolben unter Stickstoff auf 130°C erhitzt, bis eine klare, farblose Lösung entstanden ist. Die Lösung enthielt 8 Gew% CuCl-Komplex.

### Beispiel 2:

### CuCl-P(OEt)₃-Komplex in P(OEt)₃ gelöst

8.66 g (0.052 Mol) Triethylphosphit und 1.34 g (0.0135 Mol) CuCl wurden in einem Kolben unter Stickstoff auf 150°C erhitzt, bis eine klare, farblose Lösung entstanden ist. Die Lösung enthielt 35.9 Gew% CuCl-Komplex.

### Beispiel 3:

### CuCl-P(OBu)₃-Komplex in P(OBu)₃ gelöst

29.03 g (0.116 Mol) Tributylphosphit und 0.6 g (0.006 Mol) CuCl wurden in einem Kolben unter Stickstoff auf 175°C erhitzt, bis eine klare, farblose Lösung entstanden ist. Die Lösung enthielt 7.1 Gew% CuCl-Komplex.

### Beispiel 4:

### CuCl-P(OPh)₃-Komplex in P(OPh)₃ gelöst

35.99 g (0.116 Mol) Triphenylphosphit und 0.6 g (0.006 Mol) CuCl wurden in einem Kolben unter Stickstoff auf 175°C erhitzt, bis eine klare, farblose Lösung entstanden ist. Die Lösung enthielt 6.5 Gew% CuCl-Komplex.

### Beispiel 5:

### CuBr-P(OEt)₃-Komplex in P(OEt)₃ gelöst

19.3 g (0.116 Mol) Triethylphosphit und 0.86 g (0.006 Mol) CuBr wurden in einem Kolben unter Stickstoff auf 130°C erhitzt, bis eine klare, farblose Lösung entstanden ist. Die Lösung enthielt 9.2 Gew% CuBr-Komplex.

### Beispiel 6:

### CuI-P(OEt)₃-Komplex in P(OEt)₃ gelöst

19.3 g (0.116 Mol) Triethylphosphit und 1.14 g (0.006 Mol) CuI wurden in einem Kolben unter Stickstoff auf 130°C erhitzt, bis eine klare, farblose Lösung entstanden ist. Die Lösung enthielt 9.5 Gew% CuI-Komplex.

### Beispiel 7 bis 13:

### Diskontinuierliche Herstellung von Prenylchlorid

### Beispiel 7:

In einem 500 ml-Dreihalskolben mit Thermometer, Gaseinleitfritte und Rückflußkühler wurden 146 g (2.14 Mol) Isopren und 0.22 g Katalysatorlösung (17.6 mg = 0.07 mMol Komplex) aus Beispiel 1 gegeben. Nach dem Abkühlen auf 0°C wurde in ca. 90 Minuten HCl-Gas eingeleitet, bis es zum Schäumen kam, bedingt durch unvollständige HCl-Addition. Danach wurde auf Raumtemperatur erwärmt. Es wurde 217.0 g Produkt erhalten. Das Produkt wurde stehengelassen und die Umlagerung von Isoprenylchlorid zu Prenylchlorid gaschromatographisch verfolgt. Nach 10 Tagen wurde das Gleichgewicht erreicht (20 % Isoprenylchlorid, 80 % Prenylchlorid).

### Beispiel 8:

Es wurde analog Beispiel 7 vorgegangen, mit dem Unterschied, daß nur 0.02 g Katalysatorlösung (1.76 mg = 0.007 mMol Komplex) aus Beispiel 1 eingesetzt wurden. Im Vergleich zu Beispiel 7 wurde die Einleitgeschwindigkeit des Chlorwasserstoffs reduziert, so daß die Einleitdauer 3 Stunden betrug. Es wurde 208 g Produkt erhalten.
Nach der Umsetzung war die Zusammensetzung nach GC-Analyse: 29.2 % Prenylchlorid, 63.7 % Isoprenylchlorid.
Nach 9 Tagen Lagerung war die Zusammensetzung nach GC-Analyse: 64.2 % Prenylchlorid, 27.2 % Isoprenylchlorid.

### Beispiel 9:

Es wurde analog Beispiel 7 vorgegangen, mit dem Unterschied, daß 0.22 g Katalysatorlösung (79.0 mg = 0.33 mMol Komplex) aus Beispiel 2 eingesetzt wurden. Die Ausbeute betrug 219 g.
Nach der Umsetzung war die Zusammensetzung nach GC-Analyse: 35.9 % Prenylchlorid, 56.2 % Isoprenylchlorid.
Nach 2 Tagen Lagerung war die Zusammensetzung nach GC-Analyse: 80.9 % Prenylchlorid, 12.8 % Isoprenylchlorid.

### Beispiel 10:

Es wurde analog Beispiel 7 vorgegangen, mit dem Unterschied, daß 0.33 g Katalysatorlösung (23.4 mg = 0.07 mMol Komplex) aus Beispiel 3 eingesetzt wurden und die Einleitdauer von Chlorwasserstoff 50 Minuten betrug. Die Ausbeute betrug 217 g.
Nach der Umsetzung war die Zusammensetzung nach GC-Analyse: 44.0 % Prenylchlorid, 48.5 % Isoprenylchlorid.
Nach 4 Tagen Lagerung war die Zusammensetzung nach GC-Analyse: 70.5 % Prenylchlorid, 22.0 % Isoprenylchlorid.

### Beispiel 11:

Es wurde analog Beispiel 7 vorgegangen, mit dem Unterschied, daß 0.40 g Katalysatorlösung (26 mg = 0.07 mMol Komplex) aus Beispiel 4 eingesetzt wurden und die Einleitdauer von Chlorwasserstoff 95 Minuten betrug. Die Ausbeute betrug 217 g.
Nach der Umsetzung war die Zusammensetzung nach GC-Analyse: 53.5 % Prenylchlorid, 40.1 % Isoprenylchlorid.
Nach 5 Tagen Lagerung war die Zusammensetzung nach GC-Analyse: 75.7 % Prenylchlorid, 12.9 % Isoprenylchlorid.

### Beispiel 12:

Es wurde analog Beispiel 7 vorgegangen, mit dem Unterschied, daß 0.22 g Katalysatorlösung (20.24mg = 0.07 mMol Komplex) aus Beispiel 5 eingesetzt wurden. Die Ausbeute betrug 219 g.
Nach der Umsetzung war die Zusammensetzung nach GC-Analyse: 61.8 % Prenylchlorid, 31.2 % Isoprenylchlorid.
Nach 7 Tagen Lagerung war die Zusammensetzung nach GC-Analyse: 76.4 % Prenylchlorid, 13.8 % Isoprenylchlorid.

### Beispiel 13:

Es wurde analog Beispiel 7 vorgegangen, mit dem Unterschied, daß 0.23 g Katalysatorlösung (21.85 mg = 0.07 mMol Komplex) aus Beispiel 6 eingesetzt wurden und die Einleitdauer von Chlorwasserstoff 60 Minuten betrug. Die Ausbeute betrug 216 g.
Nach der Umsetzung war die Zusammensetzung nach GC-Analyse: 65.5 % Prenylchlorid, 26.1 % Isoprenylchlorid.
Nach 4 Tagen Lagerung war die Zusammensetzung nach GC-Analyse: 73.2 % Prenylchlorid, 12.8 % Isoprenylchlorid.

### Beispiel 14 und 15:

### Kontinuierliche Herstellung von Prenylchlorid:

### Beispiel 14:

Eine Tantal-Kreislaufapparatur mit Kreislaufpumpe und Kühler (Solekühlung mit -20°C) wurde mit 15 kg Isopren und 23 g Katalysatorlösung (0.03 mMol Komplex pro Mol Isopren) von Beispiel 1 befüllt. Nach Abkühlen auf 10°C wurden über 45 Minuten 6.25 m³/h HCl eingeleitet. Diese Startfüllung enthielt nach GC 4.8 % Isopren und 92.0 % Isoprenylchlorid und Prenylchlorid und 0.1 % Dichlormethylbutan. Danach wurden bei 4°C kontinuierlich 23.2 kg/h Isopren zugegeben, wobei der Isoprendosierung 1.5 g Katalysatorlösung (0.03 mMol Komplex pro Mol Isopren) aus Beispiel 1 pro kg Isopren zugemischt waren, sowie 6.9 m³/h HCl kontinuierlich eingeleitet und das Produkt kontinuierlich entnommen. Nach 17 Stunden wurden 586.5 kg Produkt erhalten. Die gaschromatische Untersuchung des Produkts ergab folgende Zusammensetzung: 5.4 % Isopren, 47.9 % Isoprenylchlorid und 43.7 % Prenylchlorid sowie 0.2 % 2,4-Dichlor-2-methylbutan. Nach Lagerung betrug der Prenylchlorid-Anteil 80.9 %.

### Beispiel 15:

Es wurde analog Beispiel 14 vorgegangen, mit dem Unterschied, daß anstelle von 6.9 m³/h HCl 7.4 m³/h HCl eingeleitet wurden. Die Produktzusammensetzung war wie folgt: 2.0 % Isopren, 47.1 % Isoprenylchlorid und 47.7 % Prenylchlorid sowie 0.3 % 2,4-Dichlor-2-methylbutan.

### Vergleichsbeispiel 1:

Es wurde analog Beispiel 7 vorgegangen, jedoch wurde kein Katalysator eingesetzt. Da die HCl-Aufnahmegeschwindigkeit ständig abnahm, mußte kontinuierlich die Einleitgeschwindigkeit reduziert werden. Die Einleitdauer betrug 3 Stunden und es wurden 217.5 g Produkt erhalten.
Nach der Umsetzung war die Zusammensetzung nach GC-Analyse:
45.3 % Prenylchlorid, 44.2 % Isoprenylchlorid.
Nach 10 Tagen Lagerung war die Zusammensetzung nach GC-Analyse:
58.6 % Prenylchlorid, 32.4 % Isoprenylchlorid.
Nach 24 Tagen Lagerung war die Zusammensetzung nach GC-Analyse:
70.4 % Prenylchlorid, 19.1 % Isoprenylchlorid.

### Vergleichsbeispiel 2:

In einem 500 ml-Dreihalskolben mit Thermometer, Gaseinleitungsfritte und Rückflußkühler wurden 146 g (2.14 Mol) Isopren und 0.53 g Cu(I)-Chlorid-Pulver gegeben. Nach Abkühlen auf 0°C wurden in ca. 90 Minuten 74 g HCl-Gas eingeleitet. Danach wurde auf Raumtemperatur erwärmt, ohne den Katalysator abzufiltrieren. Erhalten wurde ein Produkt, das nach 3 Tagen eine Zusammensetzung von 5.4 % Isopren, 76.6 % Prenylchlorid und 16.3 % Isoprenylchlorid aufwies. Vor der Verwendung mußte der Katalysator abfiltriert werden. Bei der Zugabe der ersten Tropfen einer 5 %-igen FeCl₃-Lösung in Ether kam es zu einer heftigen Reaktion.

### Testung der Aktivität der Katalysatoren bei HCl-Addition, Isomerisierung und Stabilisierung:

### Katalysatoraktivität (Addition):

Zur Bestimmung der Katalysatoraktivität bezüglich der HCl-Addition wurden konstant 2.66 l/min HCl über eine Fritte in eine Lösung aus 146 g (2.14 Mol) Isopren und der in Tabelle 1 angegebenen Katalysatormenge bei 0°C eingeleitet. Es wurde jeweils die Zeitspanne bestimmt, bis es zum Schäumen kam, da nicht mehr die Gesamtmenge HCl aufgenommen wurde. Die Geschwindigkeitsfaktoren beziehen sich jeweils auf den ohne Katalysator erhaltenen Wert. Die Ergebnisse sind in der nachfolgenden Tabelle 1 zusammengefaßt.

**Tabelle 1**

| Katalysator [Beispiel] | Menge¹ [mMol] | Zeit [s] | Geschwindigkeitsfaktor |
|---|---|---|---|
| ohne Katalysator | | 15 | 1.0 |
| CuCl-P(OEt)₃ [1] | 0.15 | 105 | 7.0 |
| CuCl-P(OEt)₃ [1] | 0.03 | 90 | 6.0 |
| CuCl-P(OBu)₃ [3] | 0.03 | 65 | 4.3 |
| CuCl-P(OPh)₃ [4] | 0.03 | 55 | 3.7 |
| CuBr-P(OEt)₃ [5] | 0.03 | 60 | 4.0 |
| CuI-P(OEt)₃ [6] | 0.03 | 45 | 3.0 |

| | | | |
|---|---|---|---|
| 1 = mMol Katalysator pro Mol Isopren | | | |

Die Messungen ergaben, daß der Komplex CuCl-P(OEt)₃ die Addition von HCl an Isopren eindeutig am besten katalysiert.

### Katalysatoraktivität (Umlagerung):

Es wurden konstant 2.66 l/min HCl über eine Fritte in eine Lösung aus 146 g (2.14 Mol) Isopren und der in Tabelle 2 angegebenen Katalysatormenge bei 0°C eingeleitet. Nach der Reaktion ließ man das Produkt von 0°C auf Raumtemperatur kommen. Bei dieser Temperatur wurde das Produkt gelagert. Die Messung des Umlagerungseffektes der Katalysatoren erfolgte durch gaschromatographische Bestimmung der Zusammensetzung in den in Tabelle 2 aufgeführten Zeitabständen. Gemessen wurde die Zusammensetzung zum Zeitpunkt 0, das heißt unmittelbar nach Beendigung des HCl-Einleitens. Außerdem wurden die Zeiten bestimmt, bei denen der Anteil an Isoprenylchlorid auf 20 % abgefallen ist bzw. der Anteil an Prenylchlorid auf 70 % gestiegen ist. Weiterhin wurde die Zeit bis zur Gleichgewichtseinstellung bestimmt, ab der keine Änderung der Zusammensetzung mehr eintritt. Die Ergebnisse der Testung sind in Tabelle 2 zusammengefaßt.

Aus den Ergebnissen in Tabelle 2 geht hervor, daß die katalytische Wirkung bezüglich der Umlagerung von der Art des Liganden ( OPh > OBu > OEt ), vom Kupferhalogenid ( CuI > CuBr > CuCl) und von der Katalysatormenge abhängt. Besonders gut wirkt der CuI-Triethylphosphit-Komplex.

**Tabelle 2**

| Katalysator [Beispiel] | Menge¹ [mMol] | Isoprenylchlorid | | Prenylchlorid | |
|---|---|---|---|---|---|
| | | Zeit [d] | Gehalt [%] | Zeit [d] | Gehalt [%] |
| ohne Katalysator | | 0 | 44.2 | 0 | 45.3 |
| | | 22.7 | 20.0 | 24.0 | 70.0 |
| CuCl-P(OEt)₃ [1] | 0.15 | 0 | 56.2 | 0 | 35.9 |
| | | 0.8 | 20.0 | 0.7 | 70.0 |
| | | 2.0 | 12.8 | 2.0 | 80.9 |
| CuCl-P(OEt)₃ [1] | 0.03 | 0 | 52.5 | 0 | 35.5 |
| | | 10.0 | 20.0 | 8.0 | 70.0 |
| | | keine Änderung | | keine Änderung | |
| CuCl-P(OEt)₃ [1] | 0.003 | 0 | 63.7 | 0 | 29.2 |
| | | 17.8 | 20.0 | 18.6 | 70.0 |
| CuCl-P(OBu)₃ [3] | 0.03 | 0 | 44.0 | 0 | 44.6 |
| | | 4.4 | 20.0 | 3.9 | 70.0 |
| | | 6.0 | 13.0 | 6.0 | 75.4 |
| CuCl-P(OPh)₃ [4] | 0.03 | 0 | 40.1 | 0 | 53.5 |
| | | 3.0 | 20.0 | 2.8 | 70.0 |
| | | 5.0 | 12.9 | 5.0 | 75.7 |
| CuBr-P(OEt)₃ [5] | 0.03 | 0 | 31.2 | 0 | 61.8 |
| | | 3.6 | 20.0 | 3.0 | 70.0 |
| | | 7.0 | 13.8 | 7.0 | 76.4 |
| CuI-P(OEt)₃ [6] | 0.03 | 0 | 26.1 | 0 | 65.5 |
| | | 1.0 | 20.0 | 1.5 | 70.0 |
| | | 4.0 | 12.8 | 4.0 | 73.2 |

| | | | | | |
|---|---|---|---|---|---|
| 1 = mMol Katalysator pro Mol Isopren | | | | | |

### Katalysatoraktivität (Stabilisierung):

Zur Bestimmung der stabilisierenden Wirkung der Katalysatoren wurden die Reaktionsprodukte der Beispiele 7 bis 13 wie folgt getestet:
10 g Rohprodukt wurden in ein Becherglas gegeben und auf 40°C erwärmt. Dann wurden 2 Tropfen einer 5 %-igen FeCl₃-Lösung in Diethylether (1 Tropfen = 0.017 g Lösung = 0.85 mg FeCl₃) zugegeben und umgeschwenkt. Wenn es nach 5 Minuten zu keiner Temperaturerhöhung kam, wurden wieder 2 Tropfen zugegeben und der Vorgang solange wiederholt, bis es zu einer stark exothermen Reaktion kam. Je höher der FeCl₃-Verbrauch pro kg Isopren, umso besser die stabilisierende Wirkung. Die Ergebnisse des Stabilitätstests sind in Tabelle 3 zusammengefaßt.

**Tabelle 3**

| Katalysator [Beispiel] | Menge¹ [mMol] | FeCl₃-Verbrauch [mg/kg] |
|---|---|---|
| CuCl-P(OEt)₃ [7] | 0.03 | 850 |
| CuCl-P(OEt)₃ [8] | 0.003 | 170 |
| CuCl-P(OEt)₃ [9] | 0.15 | 425 |
| CuCl-P(OBu)₃ [10] | 0.03 | 680 |
| CuCl-P(OPh)₃ [11] | 0.03 | 425 |
| CuBr-P(OEt)₃ [12] | 0.03 | 850 |
| CuI-P(OEt)₃ [13] | 0.03 | 425 |

Die stabilisierende Wirkung der Katalysatorlösungen steigt in der Reihe P(OPh)₃ < P(OBu)₃ < P(OEt)₃. Zur Kontrolle wurde FeCl₃, wie oben beschrieben, zu 10 g Rohprodukt aus Vergleichsbeispiel 1 gegeben. Dabei kam es bereits bei Zugabe der ersten Tropfen zu einer heftigen Reaktion.

## Patentansprüche

1. Verfahren zur Herstellung von 1-Chlor-3-methyl-but-2-en aus Isopren und Chlorwasserstoff in Gegenwart eines Katalysators, dadurch gekennzeichnet, daß als Katalysator ein oder mehrere Komplexe von Kupfer(I)-halogenid und Trialkyl- oder Triarylphosphiten der allgemeinen Formel CuX·P(OR)₃ (I), wobei X die Bedeutung Cl, Br oder I hat, und R ein geradkettiger oder verzweigter Alkylrest mit 1 bis 18 C-Atomen oder ein Arylrest ist, verwendet werden.

2. Verfahren nach Anspruch 1, dadurch gekennzeichnet, daß der Rest R ein substituierter, geradkettiger oder verzweigter Alkylrest mit 1 bis 18 C-Atomen oder ein substituierter Arylrest ist.

3. Verfahren nach Anspruch 1, dadurch gekennzeichnet, daß X = Cl, Br oder I, und R = Methyl, Ethyl, n-Propyl, iso-Propyl, n-Butyl, iso-Butyl, iso-Amyl, Benzyl oder Phenyl.

4. Verfahren nach Anspruch 1 bis 3, dadurch gekennzeichnet, daß der Katalysator der allgemeinen Formel (I) in Lösung in inerten organischen Lösungsmitteln oder in den entsprechenden Trialkylphosphiten oder Triarylphosphiten vorliegt.

5. Verfahren nach Anspruch 1 bis 4, dadurch gekennzeichnet, daß das Verfahren bei einer Temperatur von -30°C bis +30°C, bei Normaldruck oder einem Überdruck bis zu 5 bar und einem Molverhältnis von HCl zu Isopren von ≤ 1 : 1 durchgeführt wird.

6. Verfahren nach Anspruch 1 bis 5, dadurch gekennzeichnet, daß die Komplexe von Kupfer(I)-halogenid und Trialkyl- oder Triarylphosphiten der allgemeinen Formel CuX·P(OR)₃ (I) in einem Molverhältnis von 0.02 bis 2.0 mMol pro Mol Isopren eingesetzt werden.

7. Verfahren nach Anspruch 1 bis 6, dadurch gekennzeichnet, daß die Herstellung in diskontinuierlicher Fahrweise erfolgt, wobei der Katalysator zusammen mit dem Isopren vorgelegt, der Ansatz abgekühlt, anschließend unter Rühren und Kühlen Chlorwasserstoffgas in den Ansatz eingeleitet wird und nach Abschluß der HCl-Einleitung der Ansatz erwärmt und das Produkt vorzugsweise zur Isomerisierung von Isoprenylchlorid zu Prenylchlorid gelagert wird.

8. Verfahren nach Anspruch 7, dadurch gekennzeichnet, daß das Produktgemisch ohne vorhergehende Lagerung oder nach Lagerung destillativ gereinigt wird.

9. Verfahren nach Anspruch 1 bis 6, dadurch gekennzeichnet, daß die Herstellung in kontinuierlicher Fahrweise erfolgt, wobei in einen mit Kreislaufpumpe betriebenen und gekühlten Reaktionskreislauf kontinuierlich eine Isopren/Katalysatormischung oder Isopren und Katalysator getrennt sowie gasförmiger Chlorwasserstoff dosiert werden, wobei das Reaktionsprodukt kontinuierlich abdestilliert wird, das über den Kopf der Destillationskolonne abdestillierte Isopren/Isoprenylchlorid-Gemisch wieder der Reaktion zugeführt wird und das Prenylchlorid als Sumpfprodukt der Destillationskolonne gewonnen wird.

10. Verfahren nach Anspruch 9, dadurch gekennzeichnet, daß das Sumpfprodukt der Destillationskolonne einer weiteren kontinuierlichen Destillation zugeführt wird.

## Claims

1. Process for preparing 1-chloro-3-methylbut-2-ene from isoprene and hydrogen chloride in the presence of a catalyst, characterized in that the catalyst used comprises one or more complexes of copper(I) halide and trialkyl or triaryl phosphites of the general formula CuX·P(OR)₃ (I), where X is Cl, Br or I and R is a straight-chain or branched alkyl radical having from 1 to 18 carbon atoms or an aryl radical.

2. Process according to Claim 1, characterized in that the radical R is a substituted, straight-chain or branched alkyl radical having from 1 to 18 carbon atoms, or a substituted aryl radical.

3. Process according to Claim 1, characterized in that X = Cl, Br or I, and R = methyl, ethyl, n-propyl, iso-propyl, n-butyl, iso-butyl, iso-amyl, benzyl or phenyl.

4. Process according to Claim 1 to 3, characterized in that the catalyst of the general formula (I) is present in solution in inert organic solvents or in the corresponding trialkyl phosphites or triaryl phosphites.

5. Process according to Claim 1 to 4, characterized in that the process is carried out at a temperature of from -30°C to +30°C, at atmospheric pressure or super-atmospheric pressure up to 5 bar and a molar ratio of HCl to isoprene of ≤ 1 : 1.

6. Process according to Claim 1 to 5, characterized in that the complexes of copper(I) halide and trialkyl or triaryl phosphites of the general formula CuX·P(OR)₃ (I) are used in a molar ratio of from 0.02 to 2.0 mmol per mol of isoprene.

7. Process according to Claim 1 to 6, characterized in that the preparation is carried out batchwise, with the catalyst being initially charged together with the isoprene, the mixture being cooled, hydrogen chloride gas being subsequently passed into the mixture while stirring and cooling and, after passing in of HCl is complete, the mixture being warmed and the product being preferably stored to isomerize isoprenyl chloride to give prenyl chloride.

8. Process according to Claim 7, characterized in that the product mixture is purified by distillation without prior storage or after storage.

9. The process according to Claim 1 to 6, characterized in that the preparation is carried out continuously, with an isoprene/catalyst mixture or isoprene and catalyst separately and also gaseous hydrogen chloride being continuously metered into a cooled reaction circuit driven by a circulation pump, with the reaction product being continuously distilled off, the isoprene/isoprenyl chloride mixture distilled off via the top of the distillation column being recycled to the reaction and the prenyl chloride being obtained as bottom product of the distillation column.

10. Process according to Claim 9, characterized in that the bottom product of the distillation column is fed to a further continuous distillation.

## Revendications

1. Procédé de préparation de 1-chloro-3-méthylbut-2-ène à partir d'isoprène et d'acide chlorhydrique en présence d'un catalyseur, caractérisé en ce que l'on utilise, en tant que catalyseur, un ou plusieurs complexe(s) d'halogénure de cuivre (I) et de trialkyl-ou triarylphosphites de formule générale CuX·P(OR)₃ (I), dans laquelle X représente Cl, Br ou I, et R est un radical alkyle linéaire ou ramifié ayant de 1 à 18 atome(s) de carbone ou un radical aryle.

2. Procédé selon la revendication 1, caractérisé en ce que le radical R est un radical alkyle linéaire ou ramifié ayant de 1 à 18 atomes de carbone ou un radical aryle substitué.

3. Procédé selon la revendication 1, caractérisé en ce que X = Cl, Br ou I, et R = méthyle, éthyle, n-propyle, isopropyle, n-butyle, isobutyle, isoamyle, benzyle ou phényle.

4. Procédé selon les revendications 1 à 3, caractérisé en ce que le catalyseur de formule générale (I) se trouve en solution dans des solvants organiques inertes ou dans les trialkylphosphites ou triarylphosphites correspondants.

5. Procédé selon les revendications 1 à 4, caractérisé en ce que le procédé est réalisé à une température de -30°C à +30°C, à la pression normale ou à une surpression allant jusqu'à 5 bars et un rapport molaire entre HCl et isoprène ≤ 1 : 1.

6. Procédé selon les revendications 1 à 5, caractérisé en ce que les complexes d'halogénure de cuivre (I) et de trialkyl- ou triarylphosphites de formule générale CuX·P(OR)₃ (I) sont utilisés à un rapport molaire de 0,02 à 2,0 mmol par mol d'isoprène.

7. Procédé selon les revendications 1 à 6, caractérisé en ce que la préparation est réalisée de manière discontinue, le catalyseur étant chargé initialement conjointement avec l'isoprène, le mélange étant refroidi, de l'acide chlorhydrique gazeux étant ensuite introduit dans le mélange tout en agitant et en refroidissant et, à la fin de l'introduction de HCl, le mélange étant chauffé et le produit étant de préférence stocké en vue d'isomériser le chlorure d'isoprényle en chlorure de prényle.

8. Procédé selon la revendication 7, caractérisé en ce que le mélange du produit est purifié par distillation sans stockage préalable ou après stockage.

9. Procédé selon les revendications 1 à 6, caractérisé en ce que la préparation est réalisée de manière continue, en ajoutant en continu, en quantité mesurée, un mélange isoprène/catalyseur ou de l'isoprène et du catalyseur séparément, ainsi que de l'acide chlorhydrique gazeux dans un circuit de réaction refroidi, entrainé par une pompe de circulation, le produit de réaction étant distillé en continu, le mélange isoprène/chlorure d'isoprényle distillé par la tête de la colonne de distillation étant recyclé dans la réaction et le chlorure de prényle étant obtenu en tant que produit de queue de la colonne de distillation.

10. Procédé selon la revendication 9, caractérisé en ce que le produit de queue de la colonne de distillation est chargé dans une autre distillation continue.
